Europäisches Patentamt

European Patent Office

Office européen des brevets

(19)

(11) EP 0 673 238 B1

(12) **FASCICULE DE BREVET EUROPEEN**

(45) Date de publication et mention
de la délivrance du brevet:
**20.05.1998 Bulletin 1998/21**

(21) Numéro de dépôt: **94902016.8**

(22) Date de dépôt: **10.12.1993**

(51) Int. Cl.$^6$: **A61K 7/48**, A61K 7/06,
A61K 35/78

(86) Numéro de dépôt international:
**PCT/FR93/01225**

(87) Numéro de publication internationale:
**WO 94/13260 (23.06.1994 Gazette 1994/14)**

(54) **UTILISATION D'UN EXTRAIT DE SIMAROUBA POUR L'ATTENUATION DES TACHES PIGMENTAIRES DE LA PEAU**

VERWENDUNG EINES SIMAROUBAEXTRAKTES ZUR VERMINDERUNG VON FLECKIGER HAUTPIGMENTIERUNG

USE OF A SIMAROUBA EXTRACT TO REDUCE PATCHY SKIN PIGMENTATION

(84) Etats contractants désignés:
**BE CH DE ES FR GB IT LI**

(30) Priorité: **11.12.1992 FR 9214968**
**02.08.1993 FR 9309492**

(43) Date de publication de la demande:
**27.09.1995 Bulletin 1995/39**

(60) Demande divisionnaire:
**96120631.5 / 0 797 981**

(73) Titulaire: **LVMH RECHERCHE**
**92752 Nanterre (FR)**

(72) Inventeurs:
• **BONTE, Frédéric**
**F-92400 Courbevoie (FR)**
• **MEYBECK, Alain**
**F-92400 Courbevoie (FR)**
• **DUMAS, Marc**
**F-92700 Colombes (FR)**

(74) Mandataire: **Portal, Gérard et al**
**Cabinet Beau de Loménie**
**158, rue de l'Université**
**75340 Paris Cédex 07 (FR)**

(56) Documents cités:
**WO-A-91/05543**                **WO-A-92/21322**
**FR-A- 2 607 006**              **FR-A- 2 649 615**

• **DERWENT JAPANESE PATENTS REPORT 27**
**Novembre 1962 Derwent Publications Ltd.,**
**London, GB; AN 18640/65 & JP,B,40 018 640 (Y.**
**HAGIWARA) 21 Août 1965**
• **CHEMICAL ABSTRACTS, vol. 109, no. 17, 1988,**
**Columbus, Ohio, US; abstract no. 146353h, C.A.**
**ARANA ET AL. 'Picramnia macrostachys Ami**
**(Simaroubaceae)' page 434 ; & REV. PERU.**
**BIOQUIM., vol.8, no.2, 1986 pages 16 - 19**

Remarques:
Demande divisionnaire 96120631.5 déposée le
20/12/96.

**Description**

La présente invention concerne essentiellement l'utilisation d'un extrait de Simarouba pour la fabrication d'une composition cosmétique ou pharmaceutique, notamment dermatologique, présentant notamment une activité dépigmentante et une activité favorisant la différenciation des kératinocytes, et destinée en particulier à l'atténuation des taches pigmentaires cutanées, notamment des taches de sénescence, au traitement du vitiligo, au renforcement de la fonction protectrice de la peau, ou à l'amélioration de l'aspect de la chevelure et composition ainsi obtenue.

Plus particulièrement l'invention est relative à l'utilisation d'un extrait de Simarouba pour la fabrication d'une composition cosmétique ou pharmaceutique, notamment dermatologique, présentant notamment une activité dépigmentante ainsi qu'une activité favorisant la différenciation des kératinocytes, et destinée en particulier à traiter les taches pigmentaires cutanées, notamment des taches de sénescence, à traiter le vitiligo, les désordres cutanés s'accompagnant de dérèglements de la différenciation des kératinocytes, tels que le psoriasis, à restaurer, à préserver et/ou renforcer la fonction protectrice de la peau, notamment la fonction de barrière hydrique, ainsi qu'à renforcer la cohésion des cellules de l'épiderme ou encore améliorer la qualité des cheveux.

La plante Simarouba est une plante de la famille des Simaroubacées. Parmi les plantes Simarouba, on peut citer Simarouba amara Aubl., Simarouba glauca, Simarouba versicolor et Simarouba excelsa. Ces plantes sont connues pour avoir des propriétés similaires (voir le livre de Mrs M. GRIEVE ayant pour titre "Modem Herbal" édité par Mrs C. F. LEYEL aux éditions PENGUIN BOOKS, pages 741-742). Simarouba amara Aubl., en particulier, est un arbre assez répandu en Guyane, dont l'écorce est utilisée par les indigènes comme remède pour traiter la dysenterie, améliorer la tonicité de l'intestin, promouvoir les sécrétions de l'intestin et prédisposer le patient à dormir.

D'autre part, l'abrégé Chemical Abstracts 110 (02) 013 432 décrit l'utilisation d'extrait de tige de Simarouba amara pour la mise en évidence in vitro d'une activité antiamibienne. De même, la revue Chemical Abstracts, résumé 109 (101) 000285 décrit un extrait de fruit de Simarouba amara pour la préparation d'un médicament anti-paludéen.

Or, les inventeurs de la présente invention ont pu observer que les extraits de Simarouba sont particulièrement actifs au niveau des mélanocytes en ce qui concerne l'inhibition de la mélanogénèse, cette activité ayant pu être mise en évidence par des essais in vitro sur des cultures de mélanocytes, qui seront décrits plus loin. De plus, on a pu observer que les extraits de Simarouba sont également particulièrement actifs pour favoriser la différenciation des kératinocytes et peuvent être ainsi utilisés pour traiter les désordres cutanés s'accompagnant de dérèglement de la différenciation des kératinocytes. Cette différenciation se traduit en particulier, au niveau de l'épiderme, par une plus grande cohésion cellulaire, par une régulation de la transformation des kératinocytes en cornéocytes par perte du noyau et augmentation de la cornéification cellulaire, par une augmentation du nombre de couches de cornéocytes formant la couche cornée, l'ensemble de ces phénomènes contribuant au renforcement de la fonction protectrice de la peau vis-à-vis du milieu extérieur et au renforcement de la barrière hydrique empêchant la perte excessive d'eau par l'épiderme ; et, au niveau du follicule pileux, une régulation des processus de synthèse des kératines par les kératinocytes, ces protéines étant le constituant principal de la tige pilaire du cheveu et dont la qualité est essentielle pour un cheveu en bon état.

Ainsi, la présente invention a pour but de résoudre le nouveau problème technique consistant en la fourniture d'une nouvelle formulation de compositions cosmétiques ou pharmaceutiques, notamment dermatologiques ayant une bonne efficacité de dépigmentation, en rendant ainsi possible son utilisation pour réaliser l'atténuation des taches pigmentaires cutanées, en particulier des taches de sénescence, ainsi que, dans le traitement du vitiligo, l'atténuation du contraste entre les zones atteintes par le vitiligo et leur entourage. On notera, à ce propos, que les taches de sénescence résultent de phénomènes complexes, comprenant généralement une hypercoloration locale liée à une hyperpigmentation et accompagnée parfois d'une hyperkératose localisée.

La présente invention a encore pour but principal de résoudre le problème technique consistant en la fourniture d'une nouvelle formulation de composition cosmétique ou pharmaceutique, notamment dermatologique, ayant une activité favorisant la différenciation des kératinocytes et destinée en particulier à traiter les désordres cutanés s'accompagnant de dérèglement de la différenciation des kératinocytes, tels que le psoriasis, à restaurer, préserver et/ou renforcer la fonction protectrice de la peau, notamment la fonction de barrière hydrique, conduisant ainsi à un effet hydratant, en particulier en empêchant la perte excessive d'eau par l'épiderme, dont une application avantageuse est le traitement des peaux ichtyosiques ainsi que le traitement des peaux psoriasiques, et à améliorer la qualité des cheveux, embellissant ainsi l'aspect de la chevelure.

La présente invention résout pour la première fois ces problèmes techniques de manière satisfaisante et utilisable à l'échelle industrielle pour la préparation de compositions cosmétiques ou dermatologiques.

Ainsi, selon un premier aspect, la présente invention concerne l'utilisation d'un extrait de Simarouba pour la fabrication d'une composition pharmaceutique, notamment dermatologique, ou comme agent cosmétique, présentant notamment une activité dépigmentante, ainsi qu'une activité favorisant la différenciation des kératinocytes, et destinée en particulier à traiter les taches pigmentaires cutanées, notamment les taches de sénescence, à traiter le vitiligo, les désordres cutanés s'accompagnant de dérèglement de la différenciation des kératinocytes, tels que le psoriasis, à restaurer, préserver et/ou renforcer la fonction protectrice de la peau, notamment la fonction de barrière hydrique, en permettant ainsi notamment d'obtenir un effet hydratant en empêchant la perte excessive d'eau par l'épiderme, en permettant ainsi une utilisation notamment pour le traitement des peaux sèches, quel que soit le degré de sécheresse, y compris les peaux ichtyosiques, les peaux psoriasiques, à améliorer la qualité des cheveux, conduisant ainsi à un embellissement de la chevelure, ainsi qu'à renforcer la cohésion des cellules de l'épiderme.

Selon une caractéristique particulière, l'extrait précité est obtenu à partir de Simarouba amara (Aublet), Simarouba glauca, Simarouba versicolor ou Simarouba excelsa, notamment à partir des écorces de troncs, de tiges ou de racines de ces plantes.

Selon une autre caractéristique particulière, l'extrait précité est un extrait obtenu par extraction avec au moins un solvant polaire, tel que l'eau, un alcool, de préférence un alcool inférieur, tel que méthanol ou éthanol, un glycol, en particulier le propylèneglycol, ou un mélange hydro-alcoolique en toute proportion.

Selon une autre caractéristique particulière, l'extrait précité est présent dans la composition à une concentration comprise entre 0,001 et 5 % en poids, exprimée en extrait sec, par rapport au poids total de la composition, de préférence entre 0,01 et 1 % et encore de préférence entre 0,1 et 0,5 % en poids.

Selon une autre caractéristique particulière, la composition précitée contient, en outre, un agent actif choisi parmi le groupe constitué par l'acide kojique et ses sels ou esters, l'acide caféique et ses sels ou esters, l'hydroquinone et ses dérivés, un extrait de mûrier, la trétinoïne, la vitamine A ou ses dérivés ou esters, un caroténoïde, notamment le bêta-carotène, la vitamine C, un corticoïde, le glutathion, la cystéine, l'acide parahydroxycinnamique ou ses sels ou esters, les sels, esters et dérivés des substances précitées étant choisis parmi ceux cosmétiquement ou pharmaceutiquement acceptables.

Selon encore une autre caractéristique particulière, la composition précitée peut en outre contenir au moins un agent hydratant, tel que l'acide hyaluronique.

Selon une autre caractéristique particulière, l'extrait de mûrier précité est présent en une proportion en poids comprise entre 0,001 et 5 %, de préférence entre 0,01 et 1 %, encore de préférence entre 0,1 et 0,8 % en poids par rapport au poids total de la composition.

Selon encore une autre caractéristique particulière, l'acide kojique ou ses sels ou esters précités est présent dans la composition à une proportion en poids comprise entre 0,001 et 5 %, encore de préférence entre 0,01 et 2 %, en poids par rapport au poids total de la composition.

Selon une autre caractéristique particulière, l'extrait précité de Simarouba est au moins en partie incorporé dans des phases lamellaires lipidiques hydratées ou dans des liposomes, soit seul, soit combiné à au moins une autre substance active présente dans la composition.

La préparation des liposomes contenant au moins en partie l'extrait de Simarouba précité peut être réalisée selon l'un des procédés connus pour incorporer des substances actives dans des liposomes.

Selon un mode de réalisation préféré, selon la présente invention, on utilise un procédé d'atomisation des constituants de la phase lipidique, permettant d'obtenir une poudre lipidique facilement dispersible dans une solution aqueuse pour former des liposomes par exemple selon le procédé décrit dans le document US-A-4 508 703. La suspension de liposomes ainsi obtenue peut être homogénéisée au moyen d'ultrasons, ou dans le cas d'une production de masse, au moyen d'une homogénéisation sous pression, conformément au procédé décrit dans US-A-4 621 023.

L'extrait de Simarouba précité peut être incorporé soit dans la phase lipidique soit dans la phase aqueuse des liposomes en particuclier en utilisant les procédés précédemment décrits. Dans le cas de l'incorporation dans la phase lipidique des liposomes, on peut procéder comme suit. On dissout l'extrait de Simarouba avec les constituants de la phase lipidique, avant l'atomisation, dans une solution organique contenant au moins un lipide amphiphile, tel que la lécithine de soja, éventuellement un composé hydrophobe liphophile tel qu'un stérol, en particulier le cholestérol ou le β-sitostérol. De préférence, le solvant est choisi parmi le dichlorométhane, le chloroforme ou le méthanol, ou l'un de leurs mélanges.

La solution organique peut avantageusement contenir un agent anti-oxydant tel que l'α-tocophérol.

La poudre lipidique obtenue est dispersée dans un milieu aqueux convenable, par exemple une solution tampon PBS, une solution de glucose ou une solution de chlorure de sodium. On obtient ainsi une suspension de liposomes.

Selon un mode de réalisation avantageux, et surtout dans le cas d'une composition de liposomes, après avoir éventuellement homogénéisé la composition obtenue, les compositions de liposomes sont gélifiées par mélange avec un gel, tel qu'un gel de polymère vinylique, en particulier commercialisé sous la dénomination commerciale Carbopol® 940. Cette procédure de gélification est également décrite dans US-A-4 508 703, en particulier dans les exemples.

Selon un mode de réalisation avantageux de l'invention, la concentration de l'extrait de Simarouba précité est comprise entre 0,001 et 30% en poids, de préférence entre 0,01 et 10% en poids, de la phase lipidique desdits liposomes.

Selon un deuxième aspect, la présente invention concerne aussi une composition cosmétique, ou pharmaceutique, notamment dermatologique, de préférence à application topique, présentant notamment une activité dépigmentante, ainsi qu'une activité favorisant la différenciation des kératinocytes, et destinée en particulier à traiter les taches pigmentaires cutanées, notamment des taches de sénescence, à traiter le vitiligo, les désordres cutanés s'accompagnant de dérèglement de la différenciation des kératinocytes, tels que le psoriasis, à restaurer, préserver et/ou renforcer la fonction protectrice de la peau, notamment la fonction de barrière hydrique, en permettant ainsi notamment d'obtenir un effet hydratant en empêchant la perte excessive d'eau par l'épiderme, en permettant ainsi une utilisation notamment pour le traitement des peaux sèches, quel que soit le degré de sécheresse, y compris les peaux ichtyosiques, et les peaux psoriasiques, à améliorer la qualité des cheveux, conduisant ainsi à un embellissement de la chevelure, caractérisée en ce qu'elle comprend une quantité cosmétiquement ou pharmaceutiquement, notamment dermatologiquement, efficace d'un extrait de Simarouba.

D'autres caractéristiques particulières de la composition cosmétique ou pharmaceutique, notamment dermatologique, apparaissent clairement à la partir de la description précédente concernant les diverses caractéristiques particulières de l'utilisation et apparaissent également pour l'homme de l'art à partir de la description complète de l'invention, notamment illustrée par les exemples qui vont suivre.

Une composition selon l'invention précédemment décrite contenant un extrait de Simarouba, éventuellement sous forme au moins en partie incorporée dans des phases lamellaires lipidiques hydratées ou dans des liposomes, peut se présenter sous différentes formes utilisables en cosmétique ou en dermatologie. Par exemple, ces compositions peuvent être des gels, des crèmes, des laits ou des lotions.

Ces compositions, appliquées sur les zones à traiter de la peau, ou du cuir chevelu, ont pour effet de réguler la différenciation des kératinocytes, en favorisant ainsi la formation et la restauration d'un épiderme de bonne qualité, notamment au niveau de la couche cornée, de renforcer la fonction de barrière cutanée protectrice de l'épiderme, en particulier de barrière hydrique, et d'obtenir un embellissement de la chevelure, comme ceci a été expliqué plus haut en ce qui concerne les effets et les avantages de cette différenciation.

Ainsi, selon un mode de réalisation particulier, la composition cosmétique ou dermatologique selon l'invention présente un pouvoir hydratant, notamment en empêchant une perte excessive d'eau dans l'épiderme, et peut être destinée au traitement des peaux sèches, notamment des peaux ichtyosiques.

Selon un autre mode de réalisation particulier, la composition cosmétique ou dermatologique selon l'invention permet de restaurer la différenciation normale des kératinocytes lors de leur transformation en cornéocytes, accompagnée de la perte du noyau et de la cornéification cellulaire. Ainsi, cette composition peut être destinée au traitement des peaux psoriasiques.

Dans ce cadre, habituellement l'extrait de Simarouba est incorporé dans un excipient cosmétiquement ou dermatologiquement acceptable. Egalement, l'extrait de Simarouba peut être incorporé dans des phases lamellaires lipidiques hydratées ou des liposomes, comme cela a été décrit pour le premier aspect précédent.

Selon un troisième aspect la présente invention couvre encore un procédé de traitement cosmétique ou dermatologique des phénomènes d'hyperpigmentation cutanés, comprenant en particulier les taches de sénescence et autres taches pigmentaires cutanées, caractérisé en ce qu'on applique, au moins sur les taches pigmentaires cutanées à traiter, une quantité efficace d'un extrait de Simarouba pour obtenir leur atténuation. Avantageusement, cet extrait est obtenu à partir de la plante de Simarouba amara Aubl, notamment à partir des écorces. Des extraits particuliers ont été décrits dans la description précédente.

Selon un autre mode de réalisation, la présente invention concerne encore un procédé de traitement cosmétique ou dermatologique du vitiligo, caractérisé en ce qu'on applique, au moins sur les zones pigmentées de la peau avoisinant celles dépigmentées par le vitiligo, une quantité efficace d'un extrait de Simarouba pour obtenir l'atténuation de la pigmentation desdites zones pigmentées, en atténuant ainsi le contraste entre les zones pigmentées atteintes par le vitiligo et leur entourage, ce qui améliore l'aspect esthétique du sujet.

De préférence, dans les différents aspects précédents de l'invention, la concentration en poids d'extrait de Simarouba dans la composition cosmétique ou dermatologique finale, est comprise entre 0,001 et 5%, de préférence encore entre 0,01 et 1% par rapport au poids total de la composition.

D'autre part, selon un mode de réalisation particulièrement avantageux, on peut introduire dans la composition cosmétique ou dermatologique un principe actif complémentaire, en particulier un agent hydratant tel que l'acide hyaluronique.

D'autre part, dans le cadre de l'invention, lorsque la composition est une composition cosmétique, l'extrait de Simarouba précité peut être avantageusement incorporé dans un excipient cosmétiquement acceptable.

De même, lorsque cette composition est une composition pharmaceutique, notamment dermatologique, l'extrait de Simarouba précité peut être incorporé dans un excipient pharmaceutiquement, notamment dermatologiquement, acceptable.

De tels excipients sont bien connus à l'homme de l'art et résultent également de la description de plusieurs exemples de compositions qui va suivre.

Suivant une variante particulière, l'excipient de la composition cosmétique, ou celui de la composition pharmaceutique, est au moins en partie constitué de particules solides de très petite dimension, en particulier de l'ordre de 0,05 $\mu$m à 100 $\mu$m, et l'extrait de Simarouba, en tant que substance active, est distribué, au moins en partie, à la surface desdites particules, comme décrit dans la demande de brevet français FR-A-2 685 635.

Ainsi, d'autres buts, caractéristiques et avantages de l'invention apparaîtront clairement à la lumière de la description explicative ci-après faite en référence à plusieurs exemples illustratifs de l'invention qui ne sauraient donc en aucune façon limiter la portée de l'invention. Dans la présente description, incluant les exemples, les pourcentages sont donnés en poids sauf indication contraire.

## Exemple 1 de l'invention

### Extrait aqueux d'écorce de Simarouba

A partir d'un extrait d'écorce de racines de l'arbre Simarouba amara Aubl. en provenance de Guyane, on réalise une extraction avec de l'eau par extraction de type Soxhlet, c'est-à-dire au reflux pendant plusieurs heures. Le rapport solvant-écorce est en général de 10-1 en poids.

Après extraction, généralement on concentre cet extrait en vue d'une utlisation cosmétique ou pharmaceutique. L'extrait concentré est dénommé produit I1.

Naturellement, comme cela est bien compréhensible à l'homme de l'art, on peut poursuivre l'élimination du solvant, ici l'eau, jusqu'à obtention d'un extrait sec, par évaporation sous pression réduite ou par lyophilisation.

## Exemple 2 de l'invention

### Extrait méthanolique d'écorce de racines de Simarouba

A partir de 100 g d'écorce de racines de l'arbre Simarouba amara (Aubl.) en provenance de Guyane, on réalise une extraction avec 1 l de méthanol selon la méthode dite de Soxhlet, pendant plusieurs heures à reflux.

Après extraction, l'extrait méthanolique est concentré jusqu'à obtention d'un produit contenant très peu de méthanol dénommé produit I2.

## Exemple 3

### Mise en évidence de l'activité dépigmentante des extraits selon l'invention

L'activité dépigmentante des extraits selon l'invention est déterminée à différentes concentrations non cytotoxiques sur des cultures de mélanocytes de la lignée Cloudman S91 provenant de ATCC et portant la référence CCL 53.1 clone M-3. Chaque concentration d'extrait est testée sur trois boîtes de culture. Les boîtes de culture sont préparées en introduisant 200 000 cellules S91 par boîte dans un milieu de culture constitué de milieu EMEM complet contenant 2 % v/v de sérum de veau foetal et 0,08 $\mu$g/ml de mitomycine C.

Après 24 h d'incubation à 37°C sous atmosphère humide, le milieu est renouvelé par un milieu identique contenant les produits à tester mais maintenant sans mitomycine C.

5 jours d'incubation après le renouvellement du milieu, les mélanocytes sont comptés avec un appareil de comptage tel que le "Counter Coulter[®]" et le contenu en mélanine des cellules est déterminé en mesurant l'absorbance à 405 nm puis en transformant cette valeur en quantité de mélanine au moyen d'une courbe étalon établie avec de la mélanine synthétique en provenance de la société SIGMA qui établit une relation linéaire entre des quantités connues de mélanine et l'absorbance à 405 nm. La quantité de mélanine est alors ramenée à $10^6$ mélanocytes.

Parallèlement, une culture témoin de mélanocytes a été réalisée dans trois boîtes, avec la même lignée cellulaire et dans les mêmes conditions de culture, si ce n'est que le milieu de renouvellement introduit 24 h après l'incubation ne contient aucun produit à tester selon l'invention.

Les résultats obtenus en calculant pour chaque concentration d'extrait la moyenne sur les trois boîtes utilisées, sont répertoriés aux tableaux I et II pour chacun des produits I1 et I2 des exemples 1 et 2.

La significativité des résultats est déterminée par le test statistique t de Student avec un seuil de 0,05, qui compare les cultures traitées avec les produits, aux cultures témoins, non traitées.

TABLEAU I

| Produit I1 microgramme/ml | Nombre de cellules x $10^{-3}$ | Mélanine $\mu$g/$10^6$ cellules | Activité A | t |
|---|---|---|---|---|
| 0,5 | 222 ± 19 | 17 ± 1 | +63 % | S |
| 0,25 | 238 ± 3 | 27 ± 3 | +41 % | S |

TABLEAU I (suite)

| Produit I1 micro-gramme/ml | Nombre de cellules x $10^{-3}$ | Mélanine $\mu g/10^6$ cellules | Activité A | t |
|---|---|---|---|---|
| 0,1 | $234 \pm 14$ | $28 \pm 4$ | +39 % | S |
| Témoin | $236 \pm 30$ | $46 \pm 2$ | 0 | |

TABLEAU II

| Produit I2 microgramme/ml | Nombre de cellules x $10^{-3}$ | Mélanine $\mu g/10^6$ cellules | Activité A | t |
|---|---|---|---|---|
| 0,5 | $214 \pm 4$ | $29 \pm 2$ | +36 % | S |
| 0,25 | $247 \pm 16$ | $30 \pm 1$ | +34 % | S |
| 0,1 | $244 \pm 5$ | $33 \pm 2$ | +28 % | S |
| Témoin | $236 \pm 30$ | $46 \pm 2$ | 0 | |
| S = significatif. | | | | |

L'activité dépigmentante A est calculée comme suit :

$$A = \frac{T-P}{T} \times 100$$

dans laquelle

T = teneur en mélanine en microgramme pour $10^6$ cellules de l'échantillon témoin et
P = teneur en mélanine en microgramme pour $10^6$ cellules du produit testé I1 ou I2.

Il apparaît, à partir du tableau I, que le produit I1 produit une activité dépigmentante d'au moins 39 % par rapport au témoin, même à la très faible dose de 0,1 $\mu g/ml$, cette activité augmentant jusqu'à 63 % pour une dose de 0,5 $\mu g/ml$.

D'autre part, à partir du tableau II, on constate également que le produit I2 procure une activité dépigmentante d'au moins 28 % à la faible dose de 0,1 $\mu g/ml$, cette activité augmentant jusqu'à 36 % pour la dose de 0,5 $\mu g/ml$. Ainsi, les produits I1 et I2 présentent une activité dépigmentante importante.

Par ailleurs, l'activité des produits I1 et I2 est significative dès la première dose testée de 0,1 $\mu g/ml$.

Exemple 4

Test d'activité d'extrait de Simarouba sur la différenciation des kératinocytes

Pour la présente étude, on utilise un prélèvement de peau effectué lors d'une opération de chirurgie esthétique de type "lifting" sur le visage d'une femme de 61 ans.

Au jour J=-2, on ensemence 12 puits d'une plaque de culture multipuits avec $10^5$ cellules par puits de kératinocytes isolés à partir de ce prélèvement dans un milieu de culture classique pour la culture des kératinocytes, bien connu de l'homme de l'art. Avantageusement, ce milieu de culture peut être constitué par un milieu M199 additionné de 2 mM de L-glutamine, de 10% de sérum de veau foetal, de toxine cholérique, d'hydrocortisone, ainsi que du facteur de croissance épidermique EGF.

Aux jours J=0, J=4, J=7 et J=10, le milieu de culture est renouvelé par un milieu identique sur 6 des 12 puits qui constituent des puits témoins non traités, et, sur les 6 puits restants, constituant les puits traités on renouvèle avec un milieu identique, mais dans lequel on a dissous 25 $\mu g/ml$ d'extrait de Simarouba tel qu'obtenu à l'exemple 1, et sur lesquels on réalise une filtration stérilisante sur filtre, par exemple à 0,22 $\mu m$.

Au jour J=12, on réalise une numération cellulaire ainsi qu'une inclusion des cellules en résine époxy après fixation et marquage des structures cellulaires à l'acétate d'uranyle.

6

On réalise une coupe de chacun des blocs de résine ainsi obtenus sous forme de coupe ultrafine, par exemple à l'aide d'un dispositif connu sous le nom de Microtome, et on observe celle-ci au microscope électronique à transmission.

On a ensuite observé chacune des 6 coupes de la culture témoin, d'une part, et des 6 coupes de la culture traitée par l'extrait de Simarouba, d'autre part.

On a pu observer que les cellules traitées avec l'extrait de Simarouba selon l'invention, par exemple l'extrait obtenu à l'exemple 1, présentent une différenciation cellulaire nettement supérieure au témoin, à savoir :

- plus de 10 couches cellulaires alors que le témoin présente 5 couches cellulaires en moyenne et au maximum 8 ;
- 3 à 4 couches intermédiaires composées de kératinocytes ayant une abondante activité cytoplasmique, des desmosomes et des tonofilaments nombreux et réguliers, tandis que la culture témoin présente des cellules n'ayant pas ces caractéristiques, et possède une architecture non régulière ;
- les couches supérieures présentent une cornification régulière avec plusieurs couches de cornéocytes présentant une enveloppe cornée épaisse et remplie de tonofilaments, tandis que les couches supérieures des témoins possèdent peu de cornéocytes et présentent donc une cornéification très incomplète, une absence de grains de kératohyaline, la présence de restes d'organelles cellulaires, preuves d'une mauvaise différenciation.

Ces résultats nettement supérieurs sur la différenciation cellulaire obtenue avec l'extrait de Simarouba selon l'invention permet d'envisager l'application des extraits de Simarouba à la préparation de composition cosmétique ou pharmaceutique, notamment dermatologique, en particulier suivante :

- des compositions favorisant la formation d'un épiderme bien différencié donc donnant une peau "belle" ayant un grain et un toucher agréables ;
- des compositions luttant contre les troubles de la différenciation épidermique, survenant notamment au cours du vieillissement ;
- des compositions renforçant la barrière cutanée donc protégeant des agressions externes, par exemple par des allergènes ou des tensioactifs, d'une part, et limitant la perte excessive d'eau par l'épiderme, d'autre part ;
- des compositions permettant d'améliorer la qualité de la chevelure notamment par une amélioration de la qualité de la kératine de la tige pilaire du cheveu provenant de l'activité des kératinocytes des follicules qui se différencient depuis la base du poil.

Egalement, on peut envisager l'application de ces extraits à la préparation de milieux de cultures cellulaires, en particulier destinés à la culture en masse de kératinocytes, ou de compositions destinées à traiter des cultures de kératinocytes pour favoriser la vitesse et/ou la qualité de l'architecture de ces cultures lorsqu'elles sont, en particulier, destinées à fabriquer des peaux artificielles, dites équivalentes pour greffes, en particulier sur des brûlés, ou bien pour la préparation de modèles destinés à évaluer la toxicité ou la pénétration cutanée de diverses substances testées.

On donne ci-après divers exemples de compositions cosmétiques ou pharmaceutiques, notamment dermatologiques selon l'invention, utilisant un extrait de Simarouba.

Exemple 5

Composition cosmétique ou dermatologique dépigmentante sous forme de gel

| | |
|---|---|
| - extrait aqueux lyophilisé d'écorce de Simarouba amara de l'exemple 1 | 0,3 g |
| - propylèneglycol | 4 g |
| - éthanol | 3 g |
| - gel de Carbopol 940® à 2 % + conservateur          q.s.p. | 100 g |

Ce gel est utilisé en application locale 2 fois par jour pendant 6 semaines sur les zones de la peau à dépigmenter.

Exemple 6

Composition cosmétique ou dermatologique contenant un extrait de Simarouba en liposomes, sous forme de gel

On prend 0,3 g de produit I2 de l'exemple 2 que l'on dissout dans 50 ml d'un mélange dichlorométhane-méthanol 8-2 (v/v) auquel on ajoute 9 g de lécithine de soja et 1 g de cholestérol.

La solution obtenue peut être traitée pour obtenir une suspension de liposomes par le procédé d'évaporation en récipient rotatif bien connu qui consiste à déposer une couche lipidique dans le ballon de l'évaporateur rotatif par évaporation du solvant, puis ajouter de l'eau ou une solution aqueuse appropriée contenant des conservateurs, bien connus à l'homme de l'art, pour obtenir 100 g de suspension aqueuse de liposomes.

Cette suspension peut être homogénéisée au moyen d'ultrasons à la puissance de 150 W pendant 10 min à 4°C.

La suspension de liposome homogénéisée obtenue est ensuite gélifiée par addition d'un poids équivalent de gel de Carbopol 940$^®$ à 2 %, neutralisé par la triéthanolamine.

On obtient ainsi une composition gélifiée contenant 0,15 % en poids de produit I2 incorporée au moins en partie dans la bicouche lipidique des liposomes, par rapport au poids total de la composition.

Cette composition peut être appliquée localement par exemple 1 fois par jour pendant 4 semaines, en vue d'atténuer les taches de sénescence.

Exemple 7

Gel dépigmentant

| - extrait aqueux d'écorce de racines de Simarouba amara (Aubl.) selon l'exemple 1 | 0,2 |
| --- | --- |
| - extrait de murier | 0,5 |
| - acide ascorbique | 0,6 |
| - Carbopol 940$^®$ | 2,0 |
| - eau + conservateurs        q.s.p. | 100 g |

Ce gel est utilisé en applications locales sur les taches.

Exemple 8

Crème blanchissante destinée à éclaircir la peau

| - extrait méthanolique d'écorce de racines de Simarouba amara (Aubl.) selon l'exemple 2 | 0,3 |
| --- | --- |
| - Héliocarotte$^®$ | 2 |
| - huile minérale | 4,0 |
| - acide kojique | 0,5 |
| - glycéryl stéarate | 4,0 |
| - PEG 30 glycéryl stéarate | 3 |
| - eau + conservateur        q.s.p. | 100 g |

HELIOCAROTTE$^®$ se présente sous forme d'une huile titrée à 0,05 % en β-carotène. Elle est disponible commercialement auprès de la société BERTIN, Courbevoie - France.

Cette crème est utilisée en applications locales, deux fois par jour par cures de quatre semaines.

### Exemple 9

Gel dermatologique dépigmentant

| | |
|---|---|
| - extrait éthanolique d'écorce de racines de Simarouba amara (Aubl.) | 0,45 |
| - acide rétinoïque | 0,01 |
| - alcool | 30,0 |
| - Carbopol 940$^{®}$ | 2,5 |
| - eau + conservateur et antioxydant          q.s.p. | 100 g |

Les extraits éthanoliques précités ont été obtenus selon le procédé décrit à l'exemple 2, si ce n'est que l'on a remplacé le méthanol par de l'éthanol.

Ce gel est utilisé en application sur les zones foncées, deux fois par jour, pendant trois semaines.

### Exemple 10

Lait éclaircissant la peau

| | |
|---|---|
| - extrait aqueux d'écorce de racines de Simarouba amara (Aubl.) selon l'exemple 1 | 1,5 |
| - hydroquinone | 1,5 |
| - Héliocarotte$^{®}$ | 2 |
| - huile de germes de blé | 3 |
| - linoléate de tocophérol | 0,5 |
| - glycérine | 2 |
| - Carbopol | 0,5 |
| - glycéryl stéarate | 2,0 |
| - PEG 30 glycéryl stéarate | 3,0 |
| - parfums + solubilisant | 0,15 |
| - eau + conservateur          q.s.p. | 100 g |

Ce lait est utilisé en application après la douche sur les parties du corps à traiter, une fois par jour par cures de deux à trois semaines.

### Exemple 11

Composition dermatologique pour la restauration de la barrière hydrique de l'épiderme

| | |
|---|---|
| - extrait aqueux Simarouba de l'exemple 1 | 0,5 g |
| - Cremophor RH 40$^{®}$ | 1 g |
| - Carbomer 980$^{®}$ | 0,2 g |
| - triéthanolamine | 0,18 g |

(suite)

| | |
|---|---|
| - PEG 6-30 de stéarate | 7 g |
| - Cétyl alcool | 2 g |
| - huiles végétales | 25 g |
| - excipient aqueux parfumé        q.s.p. | 100 g |

On mélange les composants de manière classique pour obtenir une émulsion traitante appliquée matin et soir en massant légèrement les zones à traiter. Cette émulsion s'utilise comme crème de jour et/ou de nuit.

Exemple 12

Composition dermatologique pour le traitement de peaux psoriasiques

| | |
|---|---|
| - extrait méthanolique de Simarouba de l'exemple 2 | 0,3 g |
| - gel de Carbopol 980® à 2% | 35 g |
| - excipient aqueux parfumé        q.s.p. | 100 g |

On introduit l'extrait de Simarouba dans l'excipient aqueux pour le disperser, puis on ajoute le gel de Carbopol de manière à obtenir une composition gélifiée que l'on applique localement sur les lésions pendant 6 semaines.

Exemple 13

Composition cosmétique pour maintenir un état d'hydratation satisfaisant de l'épiderme

| | |
|---|---|
| - extrait méthanolique de Simarouba de l'exemple 2 | 0,18 g |
| - $\alpha$-bisabolol | 0,1 g |
| - acide hyaluronique | 1 g |
| - urée | 3 g |
| - excipient cosmétiquement acceptable sous forme d'un lait pour le corps        q.s.p. | 100 g |

Appliquer le lait sur les zones à traiter, en particulier sur les jambes après l'épilation. Cette composition permet en particulier de renforcer la fonction de barrière hydrique cutanée de l'épiderme par l'amélioration de la cohésion intercellulaire épidermique. Elle permet ainsi à la peau de conserver un état d'hydratation satisfaisant.

Exemple 14

Composition cosmétique liposomale pour rééquilibrer la desquamation de la couche cornée de l'épiderme, et redonner un épiderme lisse

| | |
|---|---|
| - extrait méthanolique de Simarouba de l'exemple 2 | 0,1 g |
| - lécithine de soja | 2 g |
| - $\beta$-sitostérol | 0,2 g |

(suite)

| | |
|---|---|
| - émulsion crème cosmétique à base de squalane de type huile-dans-eau, q.s.p. | 100 g |

Dans une première étape, on prépare une suspension aqueuse de liposomes encapsulant l'extrait méthanolique de Simarouba de l'exemple 2 dans la phase lipidique desdits liposomes. Pour cela, on procède de la manière suivante.

On dissout 0,1 g de l'extrait méthanolique de Simarouba de l'exemple 2,2 g de lécithine de soja et 0,2 g de β-sitostérol dans 50 ml d'un mélange 4:1 de dichlorométhane et de méthanol. Cette solution est évaporée sous pression réduite (200 mm de mercure environ) dans un ballon rotatif porté à 45°C. Le film lipidique obtenu est repris par 25 ml d'une solution aqueuse de phosphate monopotassique à 0,2 g/l et de phosphate disodique à 1,44 g/l, sous agitation pendant 1 h.

On obtient ainsi environ 25 ml d'une suspension de liposomes encapsulant l'extrait de Simarouba que l'on soumet ensuite à une homogénéisation aux ultrasons (15 mn, 150 W, 4°C).

Dans une deuxième étape, on incorpore de manière classique la suspension de liposomes obtenue à l'excipient émulsionné, pour obtenir la crème selon l'invention.

On peut appliquer quotidiennement la crème sur le visage en cas de peau rugueuse se desquamant par écailles. Cette composition renforce la cohésion de la couche cornée et régularise le détachement des cellules mortes.

Exemple 15

Composition cosmétique contenant un extrait de Simarouba, destinée au traitement préventif des peaux sèches

| | |
|---|---|
| - extrait méthanolique de Simarouba obtenu à l'exemple 2 | 0,5 g |
| - excipient émulsion crème de type huile-dans-eau, q.s.p. | 100 g |

On incorpore de manière classique l'extrait méthanolique de Simarouba de l'exemple 2 dans l'excipient émulsion crème, pour obtenir la crème selon l'invention.

On peut appliquer quotidiennement la crème sur les parties du corps que l'on souhaite traiter.

Exemple 16

Composition cosmétique contenant un extrait de Simarouba destiné au traitement des peaux ichtyosiques

| | |
|---|---|
| - glycérol | 3 g |
| - acide hyaluronique | 1 g |
| - extrait aqueux de Simarouba de l'exemple 1 | 0,2 g |
| - excipient émulsion crème de type huile-dans-eau, q.s.p. | 100g |

On incorpore de manière classique l'extrait aqueux de Simarouba obtenu à l'exemple 1 ainsi que le glycérol et l'acide hyaluronique, à l'excipient émulsion crème pour obtenir la crème selon l'invention.

On applique quotidiennement cette crème sur les zones souhaitées de la peau, jusqu'à obtention d'une peau beaucoup plus lisse grâce à un renouvellement régulier de la couche cornée.

**Revendications**

1. Utilisation d'un extrait de Simarouba pour la fabrication d'une composition pharmaceutique, notamment dermatologique, ou comme agent cosmétique, présentant une activité dépigmentante ainsi qu'une activité favorisant la différenciation des kératinocytes, et destinée à traiter les taches pigmentaires cutanées, notamment les taches de sénescence, à traiter le vitiligo, les désordres cutanés s'accompagnant de dérèglement de la différenciation des kératinocytes, tels que le psoriasis ; à restaurer, préserver et/ou renforcer la fonction protectrice de la peau, notamment la fonction de barrière hydrique, en permettant ainsi notamment d'obtenir un effet hydratant en empêchant la perte excessive d'eau par l'épiderme, en permettant ainsi une utilisation notamment pour le traitement des peaux sèches, quel que soit le degré de sécheresse, y compris les peaux ichtyosiques, les peaux psoriasiques, à améliorer la qualité des cheveux, conduisant ainsi à un embellissement de la chevelure, ainsi qu[** *WARNING! FF 19.1 **]*à renforcer la cohésion des cellules de l'épiderme.

2. Utilisation selon la revendication 1, caractérisée en ce que l'extrait précité est obtenu à partir de Simarouba amara (Aublet), Simarouba glauca, Simarouba versicolor ou Simarouba excelsa, notamment à partir des écorces de troncs, de tiges ou de racines de ces plantes.

3. Utilisation selon la revendication 1 ou 2, caractérisé en ce que l'extrait précité est un extrait obtenu par extraction avec au moins un solvant polaire, tel que l'eau, un alcool, de préférence un alcool inférieur, tel que méthanol ou éthanol, un glycol, en particulier le propylèneglycol, ou un mélange hydroalcoolique en toute proportion.

4. Utilisation selon l'une des revendications 1 à 3, caractérisée en ce que l'extrait précité est présent dans la composition à une concentration comprise entre 0,001 et 5 % en poids, exprimée en extrait sec, par rapport au poids total de la composition, de préférence entre 0,01 et 1 % et encore de préférence entre 0,1 et 0,5 % en poids.

5. Utilisation selon l'une des revendications précédentes, caractérisée en ce que la composition précitée contient, en outre, un agent actif choisi parmi le groupe constitué par l'acide kojique et ses sels ou esters, l'acide caféique et ses sels ou esters, l'hydroquinone et ses dérivés, un extrait de mûrier, la trétinoïne, la vitamine A ou ses dérivés ou esters, un caroténoïde, notamment le bêta-carotène, la vitamine C, un corticoïde, le glutathion, la cystéine, l'acide parahydroxycinnamique ou ses sels ou esters, les sels, esters et dérivés des substances précitées étant choisis parmi ceux cosmétiquement ou pharmaceutiquement acceptables.

6. Utilisation selon l'une des revendications précédentes, caractérisée en ce que la composition précitée peut en outre contenir au moins un agent hydratant, tel que l'acide hyaluronique.

7. Utilisation selon la revendication 5 ou 6, caractérisée en ce que l'extrait de mûrier précité est présent en une proportion en poids comprise entre 0,001 et 5 %, de préférence entre 0,01 et 1 %, encore de préférence entre 0,1 et 0,8 % en poids par rapport au poids total de la composition.

8. Utilisation selon la revendication 5 ou 6 caractérisée en ce que l'acide kojique ou ses sels ou esters précités est présent dans la composition à une proportion en poids comprise entre 0,001 et 5 %, encore de préférence entre 0,01 et 2 %, en poids par rapport au poids total de la composition.

9. Utilisation selon l'une des revendications précédentes, caractérisée en ce que l'extrait précité de Simarouba est au moins en partie incorporé dans des phases lamellaires lipidiques hydratées ou dans des liposomes, soit seul, soit combiné à au moins une autre substance active présente dans la composition.

10. Utilisation selon la revendication 9, caractérisée en ce que la concentration de l'extrait de Simarouba précité est comprise entre 0,001 et 30% en poids, de préférence entre 0,01 et 10% en poids, de la phase lipidique desdits liposomes.

11. Composition cosmétique à application topique, présentant notamment une activité dépigmentante, ainsi qu'une activité favorisant la différenciation des kératinocytes, et destinée en particulier à traiter les taches pigmentaires cutanées, notamment des taches de sénescence, à traiter le vitiligo, les désordres cutanés s'accompagnant de dérèglement de la différenciation des kératinocytes, tels que le psoriasis, à restaurer, préserver et/ou renforcer la fonction protectrice de la peau, notamment la fonction de barrière hydrique, en permettant ainsi notamment d'obtenir un effet hydratant en empêchant la perte excessive d'eau par l'épiderme, en permettant ainsi une utilisation notamment pour le traitement des peaux sèches, quel que soit le degré de sécheresse, y compris les peaux ichtyosiques, et les peaux psoriasiques, à améliorer la qualité des cheveux, conduisant ainsi à un embellissant de la chevelure, caractérisée en ce qu'elle comprend une quantité cosmétiquement efficace d'un extrait de Simarouba, dans un excipient cosmétiquement acceptable, ledit excipient étant au moins en partie constitué de particules solides de très petites dimensions, en particulier de l'ordre de 0,05 $\mu$m à 100 $\mu$m, et l'extrait de Simarouba, en tant que substance active, étant distribué, au moins en partie, à la surface desdites particules.

**12.** Composition selon la revendication 11, caractérisée en ce que la composition est telle que définie à l'une quelconque des revendications 2 à 9.

**13.** Procédé de traitement cosmétique des phénomènes d'hyperpigmentation cutanée, comprenant en particulier les taches de sénescence et autres taches pigmentaires cutanées, caractérisé en ce qu'on applique, au moins sur les taches pigmentaires cutanées à traiter, une quantité efficace d'un extrait de Simarouba pour obtenir leur atténuation, cet extrait étant avantageusement obtenu à partir de la plante de Simarouba citée à la revendication 2, notamment à partir des écorces.

**14.** Procédé de traitement cosmétique du vitiligo, caractérisé en ce qu'on applique au moins sur les zones pigmentées de la peau avoisinant celles dépigmentées par le vitiligo, une quantité efficace d'un extrait de Simarouba pour obtenir l'atténuation de la pigmentation desdites zones pigmentées, cet extrait de Simarouba étant avantageusement obtenu à partir de la plante telle que définie à la revendication 2, notamment à partir des écorces.

**Claims**

**1.** Use of a Simarouba extract for the manufacture of a pharmaceutical composition, especially dermatological composition, or as a cosmetic agent, which has a depigmentation activity as well as an activity promoting keratinocyte differentiation, and which is intended for treating patchy skin pigmentation, especially liver spots, treating vitiligo and skin disorders accompanied by keratinocyte differentiation disorder, such as psoriasis, restoring, preserving and/or enhancing the protective function of the skin, especially the water barrier function, thereby making it possible especially to obtain a moisturising effect by preventing excessive water loss through the epidermis, hence permitting use especially for the treatment of dry skin irrespective of the degree of dryness, including ichthyotic skin and psoriatic skin, and improving the quality of the hair, thus making it more attractive, as well as strenghthening the cohesion of the epidermal cells.

**2.** Use according to claim 1, characterised in that the above-mentioned extract is obtained from Simarouba amara (Aublet), Simarouba glauca, Simarouba versicolor or Simarouba excelsa, especially from the barks of trunks, stalks or roots of these plants.

**3.** Use according to claim 1 or 2, characterised in that the above-mentioned extract is an extract obtained by extraction with at least one polar solvent such as water, an alcohol, preferably a lower alcohol such as methanol or ethanol, a glycol, in particular propylene glycol, or an aqueous-alcoholic mixture in any proportions.

**4.** Use according to one of claims 1 to 3, characterised in that the above-mentioned extract is present in the composition at a concentration of between 0.001 and 5% by weight, preferably of between 0.01 and 1% and particularly preferably of between 0.1 and 0.5% by weight, expressed as dry extract and based on the total weight of the composition.

**5.** Use according to one of the preceding claims, characterised in that the above-mentioned composition also contains an active agent selected from the group consisting of kojic acid and salts or esters thereof, caffeic acid and salts or esters thereof, hydroquinone and derivatives thereof, a mulberry extract, tretinoin, vitamin A or derivatives or esters thereof, a carotenoid, especially beta-carotene, vitamin C, a corticoid, glutathion, cysteine, and parahydroxycinnamic acid or salts or esters thereof, the salts, esters and derivatives of the above-mentioned substances being selected from those which are cosmetically or pharmaceutically acceptable.

**6.** Use according to one of the preceding claims, characterised in that the above-mentioned composition can also contain at least one moisturiser such as hyaluronic acid.

**7.** Use according to claim 5 or 6, characterised in that the above-mentioned mulberry extract is present in a proportion by weight of between 0.001 and 5%, preferably of between 0.01 and 1% and particularly preferably of between 0.1 and 0.8% by weight, based on the total weight of the composition.

**8.** Use according to claim 5 or 6, characterised in that the above-mentioned kojic acid or salts or esters thereof is present in the composition in a proportion by weight of between 0.001 and 5% and preferably of between 0.01 and 2% by weight, based on the total weight of the composition.

**9.** Use according to one of the preceding claims, characterised in that the above-mentioned Simarouba extract is at least partially incorporated into hydrated lipidic lamellar phases or into liposomes, either by itself or in combination with at least one other active substance present in the composition.

10. Use according to claim 9, characterised in that the concentration of the above-mentioned Simarouba extract is between 0.001 and 30% by weight, preferably between 0.01 and 10% by weight, of the lipidic phase of said liposomes.

11. A cosmetic composition for topical application, which has especially a depigmentation activity as well as an activity promoting keratinocyte differentiation, and which is intended in particular for treating patchy skin pigmentation, especially liver spots, treating vitiligo and skin disorders accompanied by keratinocyte differentiation disorder, such as psoriasis, restoring, preserving and/or enhancing the protective function of the skin, especially the water barrier function, thereby making it possible especially to obtain a moisturising effect by preventing excessive water loss through the epidermis, hence permitting use especially for the treatment of dry skin irrespective of the degree of dryness, including ichthyotic skin and psoriatic skin, and improving the quality of the hair, thus making it more attractive, characterised in that said composition comprises a cosmetically effective amount of a Simarouba extract, in a cosmetically acceptable excipient, said excipient being at least partially constituted of solid particles of very small dimensions, in particular of the order of 0.05 $\mu$m to 100 $\mu$m, and the Simarouba extract, as the active substance, being at least partially distributed over the surface of said particles.

12. A composition according to claim 11, characterised in that the composition is as defined in any one of claims 2 to 9.

13. A cosmetic method of treating skin hyperpigmentation phenomena including particularly liver spots and other patchy skin pigmentation, characterised in that a Simarouba extract is applied, at least to the patchy skin pigmentation to be treated, in an effective amount for reducing it, this extract advantageously being obtained from the Simarouba plant mentioned in claim 2, especially from the barks.

14. A cosmetic method of treating vitiligo, characterised in that a Simarouba extract is applied, at least to the pigmented zones of the skin adjacent to the zones depigmented by vitiligo, in an effective amount for reducing the pigmentation of said pigmented zones, this Simarouba extract advantageously being obtained from the plant such as defined in claim 2, especially from the barks.

**Patentansprüche**

1. Verwendung eines Simaruba-Extrakts bei der Herstellung einer pharmazeutischen, insbesondere dermatologischen Zusammensetzung, oder als Kosmetikum, mit depigmentierender Wirksamkeit sowie einer die Differenzierung von Keratinozyten fördernden Wirksamkeit, und zur Behandlung von Hautpigmentflecken, insbesondere Altersflecken, zur Behandlung von Vitiligo, Hautstörungen, die von einer Deregulierung der Differenzierung von Keratinozyten begleitet sind, wie Psoriasis; zur Wiederherstellung, Aufrechterhaltung und/oder Verstärkung der Schutzfunktion der Haut, insbesondere der Funktion als Feuchtigkeitsbarriere, wobei so ermöglicht wird, insbesondere eine Hydratisierungswirkung durch die Verhinderung eines übermäßigen Wasserverlusts der Epidermis zu erhalten, und so eine Verwendung insbesondere zur Behandlung trockener Haut, ungeachtet des Trockenheitsgrades, ermöglicht wird, einschließlich ichtyosischer Haut und psoriatischer Haut, zur Verbesserung der Haarqualität, was so zu einer Verschönerung des Haarwuchses sowie zur Verstärkung der Kohäsion der Zellen der Epidermis.

2. Verwendung nach Anspruch 1, dadurch gekennzeichnet, daß der Extrakt aus Simaruba amara (Aublet), Simaruba glauca, Simaruba versicolor oder Simaruba excelsa, insbesondere aus den Rinden von Stämmen, Stielen oder Wurzeln dieser Pflanzen, erhalten wird.

3. Verwendung nach Anspruch 1 oder 2, dadurch gekennzeichnet, daß der Extrakt ein Extrakt ist, der durch Extraktion mit zumindest einem polaren Lösungsmittel, wie Wasser, einem Alkohol, vorzugsweise einem niederen Alkohol, wie Methanol oder Ethanol, einem Glykol, insbesondere Propylenglykol, oder einer hydroalkoholischen Mischung in einem beliebigen Verhältnis erhalten wird.

4. Verwendung nach einem der Ansprüche 1 bis 3, dadurch gekennzeichnet, daß der Extrakt in der Zusammensetzung in einer Konzentration zwischen 0,001 und 5 Masse-%, ausgedrückt als Trocken-Extrakt, bezogen auf die Gesamtmasse der Zusammensetzung, vorzugsweise zwischen 0,01 und 1 Masse-% und bevorzugter zwischen 0,1 und 0,5 Masse-% vorliegt.

**5.** Verwendung nach einem der vorhergehenden Ansprüche, dadurch gekennzeichnet, daß die Zusammensetzung außerdem ein aktives Mittel, ausgewählt aus der Gruppe bestehend aus Kojisäure und ihren Salzen oder Estern, Coffeinsäure und ihren Salzen oder Estern, Hydrochinon und seinen Derivaten, einem Brombeer-Extrakt, Tretinoin, Vitamin A oder seinen Derivaten oder Estern, Carotinoid, insbesondere β-Carotin, Vitamin C, einem Corticoid, Glutathion, Cystein, p-Hydroxyzimtsäure oder ihren Salzen oder Estern, Salzen, Estern und Derivaten der vorgenannten Substanzen, die unter den kosmetisch oder pharmazeutisch annehmbaren ausgewählt werden, enthält.

**6.** Verwendung nach einem der vorhergehenden Ansprüche, dadurch gekennzeichnet, daß die Zusammensetzung außerdem zumindest ein Hydratisierungsmittel, wie Hyaluronsäure, enthalten kann.

**7.** Verwendung nach Anspruch 5 oder 6, dadurch gekennzeichnet, daß der Brombeer-Extrakt in einer Massemenge zwischen 0,001 und 5 Masse-%, vorzugsweise zwischen 0,01 und 1 Masse-%, bevorzugter zwischen 0,1 und 0,8 Masse-%, bezogen auf die Gesamtmasse der Zusammensetzung, vorliegt.

**8.** Verwendung nach Anspruch 5 oder 6, dadurch gekennzeichnet, daß die Kojisäure oder ihre Salze oder Ester in der Zusammensetzung in einer Massemenge zwischen 0,001 und 5 Masse-%, bevorzugter zwischen 0,01 und 2 Masse-%, bezogen auf die Gesamtmasse der Zusammensetzung, vorliegt.

**9.** Verwendung nach einem der vorhergehenden Ansprüche, dadurch gekennzeichnet, daß der Simaruba-Extrakt zumindest teilweise in hydratisierte lamellare Lipidphasen oder in Liposomen, entweder allein oder kombiniert mit zumindest einer weiteren aktiven Substanz, die in der Zusammensetzung vorliegt, eingeschlossen wird.

**10.** Verwendung nach Anspruch 9, dadurch gekennzeichnet, daß die Konzentration des Simaruba-Extakts zwischen 0,001 und 30 Masse-%, vorzugsweise zwischen 0,01 und 10 Masse-%, der Lipidphase der Liposomen vorliegt.

**11.** Kosmetische Zusammensetzung zur topischen Verwendung, insbesondere mit depigmentierender Wirksamkeit sowie einer die Differenzierung von Keratinozyten fördernden Wirksamkeit, und insbesondere zur Behandlung von Hautpigmentflecken, insbesondere Altersflecken, zur Behandlung von Vitiligo, Hautstörungen, die von einer Deregulierung der Differenzierung von Keratinozyten begleitet sind, wie Psoriasis, zur Wiederherstellung, Aufrechterhaltung und/oder Verstärkung der Schutzfunktion der Haut, insbesondere der Funktion als Feuchtigkeitsbarriere, wobei so ermöglicht wird, insbesondere eine Hydratisierungswirkung durch die Verhinderung eines übermäßigen Wasserverlusts der Epidermis zu erhalten, und so eine Verwendung insbesondere zur Behandlung trockener Haut, ungeachtet des Trockenheitsgrades, ermöglicht wird, einschließlich ichtyosischer Haut und psoriatischer Haut, zur Verbesserung der Haarqualität, was so zu einer Verschönerung des Haarwuchses führt, dadurch gekennzeichnet, daß sie eine kosmetisch wirksame Menge eines Simaruba-Extrakts in einem kosmetisch annehmbaren Exzipienten umfaßt, wobei der Exzipient zumindest teilweise aus festen Teilchen mit sehr kleinen Abmessungen, insbesondere in der Größenordnung von 0,05 μm bis 100 μm, besteht, und der Simaruba-Extrakt als aktive Substanz zumindest teilweise an der Oberfläche der Teilchen verteilt ist.

**12.** Zusammensetzung nach Anspruch 11, dadurch gekennzeichnet, daß die Zusammensetzung wie in einem der Ansprüche 2 bis 9 definiert ist.

**13.** Verfahren zur kosmetischen Behandlung von Hauthyperpigmentationserscheinungen, die insbesondere Altersflecken und andere Hautpigmentflecken umfassen, dadurch gekennzeichnet, daß zumindest auf den zu behandelnden Hautpigmentflecken eine wirksame Menge eines Simaruba-Extrakts aufgebracht wird, um ihre Abschwächung zu erhalten, wobei dieser Extrakt vorteilhaft aus der in Anspruch 2 angegebenen Simaruba-Pflanze, insbesondere aus den Rinden, erhalten wird.

**14.** Verfahren zur kosmetischen Behandlung von Vitiligo, dadurch gekennzeichnet, daß zumindest auf den pigmentierten Hautzonen, die an die durch Vitiligo depigmentierten Hautzonen angrenzen, eine wirksame Menge eines Simaruba-Extrakts aufgebracht wird, um die Abschwächung der Pigmentierung der pigmentierten Zonen zu erhalten, wobei dieser Simaruba-Extrakt vorteilhaft aus der wie in Anspruch 2 definierten Pflanze, insbesondere aus den Rinden, erhalten wird.